# EUROPÄISCHE PATENTSCHRIFT

(11) **EP 1 970 034 B1**
(45) Veröffentlichungstag und Bekanntmachung des Hinweises auf die Patenterteilung: **23.12.2009**
(21) Anmeldenummer: 07005280.8
(22) Anmeldetag: 14.03.2007
(51) Int. Cl.: A61F 9/013

(54) **Apparat zur Ankopplung eines Elements an das Auge**
Apparatus for connecting an element to an eye
Appareil destiné au couplage d'un élément sur l'oeil

(43) Veröffentlichungstag der Anmeldung: 17.09.2008
(60) Teilanmeldung: 09012579.0
(73) Patentinhaber: WaveLight AG, 91058 Erlangen (DE)
(72) Erfinder: Donitzky, Christof, 90542 Eschenau (DE); Wüllner, Christian, 91096 Möhrendorf (DE); Mrochen, Michael, Dr., 8002 Zürich (CH); Büeler, Michael, Dr., 8002 Zürich (CH)
(74) Vertreter: von Hellfeld, Axel

(56) Entgegenhaltungen:
- EP-A- 1 199 055
- EP-A1- 0 327 693
- WO-A-03/002008
- WO-A-2004/096106
- WO-A-2005/048895
- WO-A-2006/090217
- WO-A-2006/121066
- US-A- 4 546 773
- US-A- 6 126 668
- US-A1- 2005 192 562

## Beschreibung

Die Erfindung betrifft eine Vorrichtung für die ophthalmologische Chirurgie.

Gepulste Laserstrahlung wird in der Augenchirurgie beispielsweise zum Anbringen von Schnitten in der Hornhaut (Kornea) oder zum Abtrag (Ablation) von Gewebe aus der Hornhaut verwendet. Die eingestrahlte Laserstrahlung bewirkt im Hornhautgewebe einen photodisruptiven bzw. photoablativen Prozess, der zur Gewebetrennung bzw. zur Entfernung von Gewebematerial führt. Derartige Bearbeitungen der Kornea finden beispielsweise im Rahmen von refraktiven Verfahren zur Minderung oder vollständigen Behebung von Fehlsichtigkeiten des Auges statt, bei denen die Kornea neu geformt wird und hierdurch ihre Brechungseigenschaften verändert werden.

Das dominierende refraktive Verfahren der Hornhautchirurgie ist die sogenannte LASIK (Laser in situ Keratomileusis). Hierbei wird aus der Hornhaut entweder mechanisch (mittels einer oszillierenden Schneidklinge in einem sogenannten Mikrokeratom) oder optisch (mittels Laserstrahlung, z.B. mittels Femtosekunden-Lasersysteme) ein kleiner Deckel herausgeschnitten, der mit einem Teil seines Randes noch an der Hornhaut hängt. Anschließend wird dieser üblicherweise auch als Flap bezeichnete Deckel zur Seite geklappt, wodurch das darunter liegende Stroma zugänglich wird. Mit Laserstrahlung wird dann nach Maßgabe eines für den jeweiligen Patienten ermittelten Ablationsprofils Stromagewebe abgetragen. Der Deckel wird danach wieder zurückgeklappt, wodurch die Wunde relativ schnell verheilen kann.

Für eine präzise Einkopplung der Laserstrahlung in das Auge ist hierbei bekannt, das Auge mittels einer Fixationsvorrichtung zu fixieren, welche durch Unterdruck am Auge angesaugt wird. Die Fixationsvorrichtung kann ein als Einkoppelelement für die Laserstrahlung dienendes Glas aufweisen. Derartige Fixationsvorrichtungen werden auch als Saugringe bezeichnet.

Sobald der Saugring am Auge eines Patienten angeordnet und mittels eines Unterdruckes an dem Auge fixiert ist, wird mittels Impulsen eines Femtosekunden-Lasers Energie in das Hornhautinnere eingebracht. Dadurch wird in der Hornhaut ein Schnitt erzeugt, der Flap kann aufgeklappt werden und die Korrektur der Fehlsichtigkeit kann durch einen definierten Abtrag von freigelegtem Hornhautgewebe erfolgen.

Saugringe per se sind dem Fachmann bekannt, beispielsweise offenbaren die US 5 336 215 und US 5 549 632 Saugringe, die in ihrem Umfangsbereich als Saugbereiche ausgebildete Öffnungen zum Ansaugen an ein Auge umfassen. Die EP 0 993 814 A1 sowie die US 6 342 053 B1 offenbaren Saugringe, bei denen ein Unterdruck im Bereich einer Applanationsfläche erzeugt wird, so dass die Hornhaut des Auges daran anliegt. Die US 6 344 040 B1 zeigt einen Saugring, bei dem ein Unterdruck im Bereich einer Applanationsfläche erzeugt wird, wobei der Saugring ferner eine Sonde umfasst, die im Einsatz die Hornhaut durchsticht und mittels einer Absaugung die während des photodisruptiven Verfahrens entstehenden Gase und Partikel absaugt. Die WO 03/002008 A1 offenbart einen Saugring mit einem am Umfang des Saugringes ausgebildeten Ansaugbereich, wobei an dem Saugring mittels eines zangenartigen Greifers eine konusförmige Linsenhalterung mit einer Linse angeordnet wird.

Die WO 00/41660 A1 beschreibt eine Einrichtung zum Durchführen einer Operation am Auge mit einem ersten ringförmigen, festen Vakuumbereich und einem zentralen, beweglichen Vakuumbereich. Der bewegliche Vakuumbereich befindet sich im Einsatz über der zu operierenden Hornhaut und kann dieser eine für die Operation gewünschte Form geben. Der zweite Vakuumbereich kann eine Mehrzahl von Elementen umfassen, so dass dessen Form und folglich die Kontur der Hornhaut während der Operation verändert werden kann.

Die WO 03/001991 A1 offenbart eine Kontaktlinse mit einer Mehrzahl von Dehnungsmesseinrichtungen zum Messen des Augeninnendruckes. Die Stromversorgung der Dehnungsmesseinrichtungen und die Kommunikation erfolgen berührungslos.

WO 2004/096106 A1 befasst sich mit einem mechanischen Mikrokeratom zur Anbringung von Schnitten in der Kornea eines Auges. Das Mikrokeratom weist einen am Auge fixierbaren Saugring auf, mit dem eine Aufnahme für einen Applanationsstempel schraubgekoppelt ist. Der Stempel ist mittels Unterdruck in der Aufnahme gehalten.

EP 1 199 055 A1 zeigt in seiner Figur 4 ebenfalls ein mechanisches Mikrokeratom mit einer Schneidklinge zum Schneiden eines Flaps in der Kornea. Das Mikrokeratom besitzt einen als Positionierring bezeichneten Saugring sowie einen Träger, an dem ein zum Durchschauen nutzbares Kontaktglas gehalten ist, das mit seiner Unterseite für eine Einebnung der Kornea sorgt. Sowohl der Saugring als auch der Träger sind jeweils durch eine Saugkammer am Auge festgesaugt. Der Saugring und der Träger sind fest miteinander verbunde. Beim Aufsetzen des Mikrokeratoms auf das Auge drückt das Kontaktglas gegen die Kornea und ebnet diese ein. Anschließend werden die beiden Saugkammern evakuiert.

US 4,546,773 betrifft eine Messsonde mit einem Ultraschallwandler zur Messung der Korneadicke.

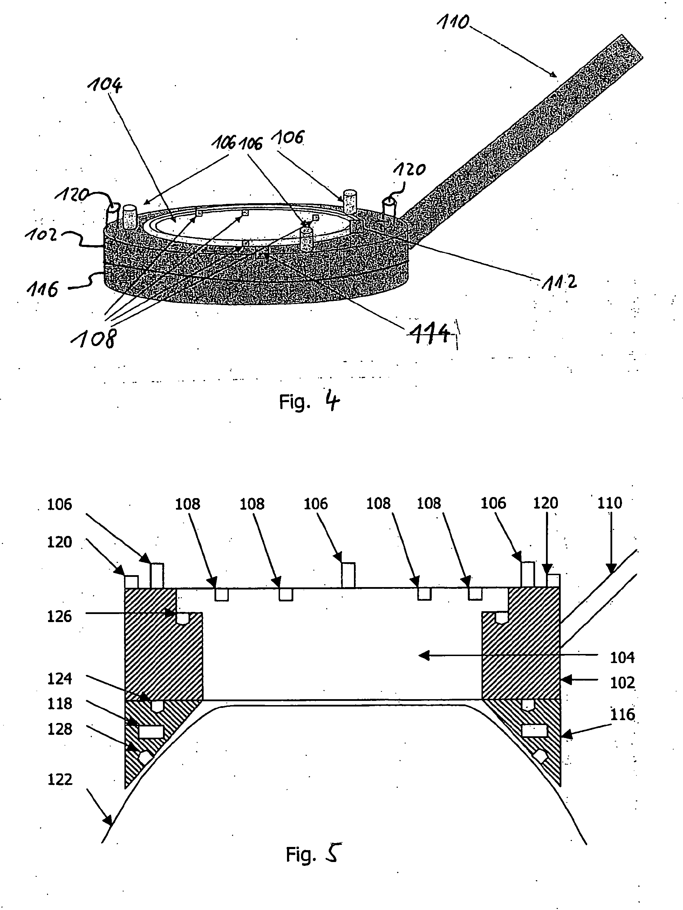

WO 2006/090217 A1 befasst sich mit einer Vorrichtung zur Ankopplung eines zu behandelnden Auges an ein Lasersystem, wobei gemäß dem Ausführungsbeispiel der Figur 4 die Vorrichtung einen sich trichterförmig erweiternden Saugring aufweist, der mit einem mit dem Lasersystem gekoppelten konischen Adapter in Eingriff bringbar ist. Der Saugring trägt eine Linse, an dessen Unterseite sich die Hornhaut des Auges anschmiegt.

WO 2006/121066 A1 ist mit einem Kühlaufsatz für ein mittels Laserstrahlung behandeltes Auge befasst. Der Kühlaufsatz wird auf das Auge aufgesetzt, ohne dabei an das Auge angesaugt zu werden. Er enthält eine unmittelbar zur Augenoberfläche hin offene und durch ein Kontaktelement vom Auge getrennte Kühlkammer, welche mit einem Kühlfluid befüllt wird.

EP 0 327 693 A1 beschreibt ein Gerät zur Untersuchung des Augenhintergrunds und Bestimmung des Augeninnendrucks. Das Gerät umfasst einen Andruckkörper mit einer konkav gewölbten Andruckfläche, mit welcher er auf das Auge aufgesetzt wird. Der Andruckkörper ist mit verschiedenen Sensoren bestückt, welche ihre Messdaten an eine Auswerteschaltung liefern.

US 6,126,668 betrifft wiederum ein mechanisches Mikrokeratom mit einem Saugring und einer auf das Auge aufzudrückenden Applanationsplatte.

WO 2005/048895 A1 zeigt in ihrer Figur 7 einen Adapter zur Ankopplung eines Auges an ein Lasersystem. Der Adapter besitzt ein in einen Halter eingeklebtes Kontaktglas zur Anlage an der Hornhaut des Auges. Der Halter weist einen abstehende Ringfortsatz auf, der beim Aufsetzen des Kontaktglases auf das Auge zuerst in Kontakt mit der Augenoberfläche kommt. Eine zwischen dem Ringfortsatz und der Unterseite des Kontaktglases begrenzte Kammer ist über einen Stutzen evakuierbar. Hierdurch ist der Halter mit dem Kontaktglas am Auge festsaugbar.

US 2005/0192562 A1 zeigt ein Augenpositioniersystem, das dem aus WO 2006/090217 A1 entspricht.

Erfindungsgemäß ist in Übereinstimmung mit Anspruch 1 eine Vorrichtung für die Augenchirurgie vorgesehen, umfassend einen auf ein zu behandelndes Auge aufsetzbaren Saugring mit einem ersten Saugbereich, der dazu ausgebildet ist, durch Unterdruckerzeugung in dem ersten Saugbereich den Saugring an das Auge anzusaugen, und ein an den Saugring ankoppelbares Funktionselement mit einem optischen Element zur Anlage der Hornhaut des Auges. Die Neuerung besteht hierbei darin, dass der Saugring einen weiteren Saugbereich aufweist, welcher dazu augebildet ist, durch Unterdruckerzeugung in dem weiteren Saugbereich das Funktionselement an den Saugring anzusaugen und die Hornhaut an das optische Element anzusaugen.

Bevorzugte Weiterbildungen dieses Erfindungsgedankens finden sich in den abhängigen Ansprüchen 2 bis 13.

Der Saugbereich kann beispielsweise durch eine Öffnung oder eine Ausnehmung gebildet sein, in der im Einsatz ein Unterdruck herrscht. Der Unterdruck kann beispielsweise durch eine mit dem Saugbereich verbundene Saugpumpe erzeugt werden. Es ist jedoch auch möglich, dass der Unterdruck beim Ankoppeln des Funktionselementes an den Saugring oder beim Ankoppeln des Saugrings an das Auge automatisch entsteht, indem beispielsweise eine Dichtlippe verschoben wird, so dass ein evakuierter Saugbereich entsteht.

Das Funktionselement umfasst ein optisches Element, beispielsweise ein Glas oder eine Linse, durch welche die Laserstrahlung in die Hornhaut eingebracht wird. Das optische Element kann eine Linse oder eine Applanationsplatte sein. Das Funktionselement kann ein Halterungselement sein, das dazu ausgebildet ist, dass weitere Elemente daran angeordnet werden können. Das optische Element kann an dem Halterungselement angeordnet sein. Dadurch entsteht eine besonders vielseitig einsetzbare Vorrichtung, da das optische Element sehr einfach ausgewechselt werden kann. Das Funktionselement kann dazu ausgebildet sein, mit einer optischen Einrichtung gekoppelt zu werden. Die optische Einrichtung kann ein Lasersystem, beispielsweise ein Femtosekunden-Lasersystem, mit einer dazugehörigen Optik sein. Das Funktionselement kann auch ein eingangs genanntes mechanisches Mikrokeratom sein. Das Funktionselement kann auch ein Adaptionskegel sein, mit dem der Saugring an eine ophthalmologische Einrichtung gekoppelt wird. Das Funktionselement kann sowohl zur Halterung eines Applanationselementes als auch zum Koppeln der Augensaugeinrichtung mit der ophthalmologischen Einrichtung vorgesehen sein. Im Folgenden wird ein derartiges Funktionselement als Halterungselement bezeichnet.

Die Vorrichtung kann eine erste Unterdruckzuführung, die mit dem ersten Saugbereich verbunden ist, und eine dritte Unterdruckzuführung, die mit einem dritten Saugbereich verbunden ist, umfassen. Der dritte Saugbereich ist an dem Saugring oder dem Funktionselement angeordnet und ist dazu ausgebildet, im Einsatz das Funktionselement an den Saugring zu saugen. Die Unterdruckzuführungen können an eine oder mehrere Saugpumpen angeschlossen sein. Im Einsatz kann in der ersten Unterdruckzuführung ein anderer Unterdruck herrschen als in der dritten Unterdruckzuführung, wodurch sich unterschiedliche Unterdrücke im ersten und im dritten Saugbereich ergeben. Dadurch ist es möglich, das Funktionselement sicher mit der Augensaugeinrichtung zu koppeln, ohne dass dabei im ersten Saugbereich ein so hoher Unterdruck erzeugt wird, dass das Auge verletzt werden könnte.

Die Vorrichtung kann derart ausgebildet sein, dass im Einsatz zumindest ein Bereich der Hornhaut eines Auges an dem Funktionselement und/oder an einem daran angeordneten Element anliegt. Dadurch ergibt sich eine genaue Fixierung der Hornhaut, was einen sicheren Operationsvorgang sicherstellt. Der weitere Saugbereich steht im Einsatz mit der Oberfläche des Funktionselementes und/oder mit der Oberfläche des daran befestigen Elementes, an dem/denen im Einsatz die Hornhaut des Auges anliegt, in Fluidverbindung . Dadurch wird die Hornhaut besonders gut in ihrer Position festgelegt, da der Bereich zwischen der Hornhaut und dem Funktionselement bzw. einem an dem Halterungselement angeordneten Element evakuiert wird bzw. unter einem Unterdruck steht. Ferner ist es möglich, den in dem Bereich zwischen der Hornhaut und dem Funktionselement und/oder einem daran angeordneten Element herrschenden Unterdruck unabhängig vom Unterdruck im ersten und dritten Saugbereich einzustellen. Dadurch wird die Verletzungsgefahr minimiert und/oder der Behandlungskomfort für einen Patienten erhöht, da sich die Kraft, mit der die Augensaugeinrichtung an das Auge gesaugt wird, von der Kraft unterscheiden kann, mit der die Hornhaut an das Funktionselement und/oder an ein daran angeordnetes Element gesaugt wird. Zudem entsteht auch eine besonders sichere Vorrichtung, die eine Redundanz aufweist, da zwei Unterdrucksysteme verwendet werden, um einerseits den Saugring am Auge zu fixieren und andererseits die Hornhaut an einem Funktionselement und/oder an einem daran angeordneten Element zu fixieren.

Bei einer vereinfachten Ausführungsform kann die Unterdruckzuführung des weiteren Saugbereichs mit der ersten oder der dritten Unterdruckzuführung in Fluidverbindung stehen.

Die Vorrichtung kann zumindest ein Messmittel umfassen. Der Ausdruck Messmittel umfasst in diesem Kontext auch das qualitative und/oder quantitative Bestimmen oder Ermitteln einer geometrischen, physikalischen und/oder chemischen Größe.

Zumindest eines der Messmittel der Vorrichtung kann dazu ausgebildet sein, eine Messung bezüglich einer Eigenschaft des Auges durchzuführen. Das Messmittel kann dazu ausgebildet sein, den Augeninnendruck zu bestimmen, wobei der Augeninnendruck beispielsweise mittels taktiler, mechanischer, akustischer und optischer Verfahren, insbesondere auch Resonanzverfahren, ermittelt werden kann.

Zumindest ein Messmittel der Vorrichtung kann dazu ausgebildet sein, Eigenschaften der Hornhaut des Auges zu messen. Das Messmittel zum Messen der Eigenschaften der Hornhaut des Auges kann den Wassergehalt der Hornhaut, eine biomechanische Eigenschaft der Hornhaut und/oder die Transparenz der Hornhaut messen. Der Wassergehalt kann beispielsweise mittels eines optischen Spektrometers bestimmt werden, die biomechanischen Eigenschaften der Hornhaut können beispielsweise durch mechanische Spektroskopieverfahren bestimmt werden und die Transparenz der Hornhaut kann durch Lichtstreuung bestimmt werden.

Die Vorrichtung kann ein Messmittel zum Messen einer auf das Auge wirkenden Größe umfassen. Das Messmittel zum Messen einer auf das Auge wirkenden Größe kann eine auf das Auge wirkende Kraft messen. Dazu können Drucksensoren, beispielsweise piezoelektrische Drucksensoren, oder Kraftsensoren in den Saugring integriert werden. Ferner ist es möglich, mikroelektromechanische Systeme (MEM-Systeme) zu verwenden, die beispielsweise an dem optischen Element angeordnet sind, über das die Laserstrahlung eingebracht wird.

Die Vorrichtung kann zumindest ein Messmittel zur Messung einer Eigenschaft des Auges und/oder der Augensaugeinrichtung bezüglich der Umgebung umfassen. Das Messmittel zum Messen einer Eigenschaft des Auges und/oder der Augensaugeinrichtung bezüglich der Umgebung kann beispielsweise die Position des Auges und/oder der Augensaugeinrichtung im Raum messen. Das Mittel zum Messen der Eigenschaft des Auges und/oder des Saugringes gegenüber der Umgebung kann dazu ausgelegt sein, mit Positionierungsmitteln für eine Behandlungsliege und/oder Positionierungsmitteln für Laserstrahlung zusammenzuwirken. Dadurch kann sichergestellt werden, dass sich das Auge immer in der korrekten Position befindet und die Laserstrahlung an der korrekten Position und unter dem korrekten Winkel auf die Hornhaut des Auges auftrifft. Die Messung der Position des Auges und/oder des Saugringes kann auf einer mechanischen, hochfrequenzbasierten, akustischen oder (dreidimensionalen) optischen Positionserkennung beruhen. Die Messmittel können an einem Funktionselement angeordnet sein, das an den Saugring gesaugt wird.

Die Messmittel können dazu ausgelegt sein, ermittelte Messdaten zu übertragen. Die Messdaten können induktiv, über ein Kabel, über eine optische Schnittstelle oder über eine elektromagnetische Schnittstelle übertragen werden. Das Messmittel kann eine Batterie umfassen, induktiv mit Strom versorgt werden oder über eine Zuleitung mit Strom versorgt werden. Das Messmittel kann ferner eine Kommunikationseinrichtung umfassen. Beispielsweise kann das Messmittel als Transponder ausgebildet sein, so dass von außen eine induktive oder elektromagnetische Anregung erfolgt und die Kommunikationseinrichtung des Messmittels die ermittelten Messwerte induktiv oder elektromagnetisch überträgt.

Die Erfindung wird nachfolgend anhand der beigefügten Zeichnungen näher erläutert. Es stellen dar:
Figur 1 einen schematischen, nicht maßstabsgetreuen Schnitt durch eine erste Ausführungsform der Erfindung;
Figur 2a einen schematischen, nicht maßstabsgetreuen Schnitt durch eine zweite Ausführungsform der Erfindung;
Figur 2b einen schematischen, nicht maßstabsgetreuen Schnitt durch eine dritte Ausführungsform der Erfindung;
Figur 3 eine schematische Frontansicht der Erfindung;
Figur 4 eine perspektivische, nicht maßstabsgetreue Ansicht eines Vergleichsbeispiels, das einem Saugring mit einer Messeinrichtung umfasst; und
Figur 5 einen schematischen, nicht maßstabsgetreuen Schnitt durch das Vergleichsbeispiel.

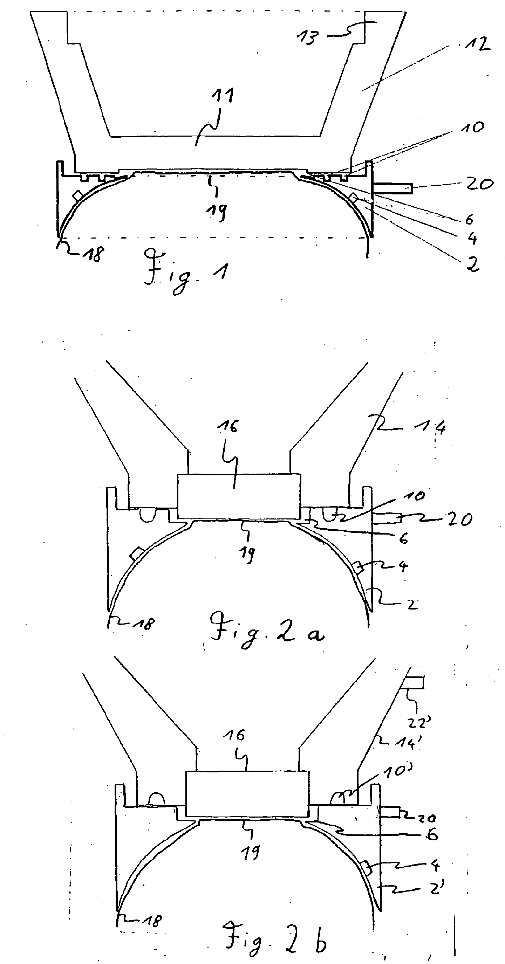

Figur 1 zeigt einen Saugring 2 mit einem Saugbereich 4, der dazu ausgebildet ist, im Einsatz den Saugring 2 an ein Auge 18 zu saugen, und einem Saugbereich 10, der dazu ausgebildet ist, im Einsatz ein Funktionselement 12 anzusaugen. Das Funktionselement 12 umfasst ein optisches Element 11, das als Linse oder Platte 11 ausgebildet ist. Ferner umfasst das Funktionselement 12 einen Anschlussbereich 13, mit dem das Funktionselement an eine optische Einrichtung (nicht gezeigt) eines Lasersystems, beispielsweise eines Femtosekunden-Lasersystems, gekoppelt werden kann. Der Saugbereich 4 bildet einen ersten Saugbereich im Sinne der Erfindung.

Ferner umfasst der Saugring 2 einen Saugbereich 6, der ein weiterer Saugbereich im Sinne der Erfindung ist und mit dem Raum zwischen dem Funktionselement 12 und der Hornhaut 19 des Auges 18 in Fluidverbindung steht und diesen zumindest teilweise evakuiert, so dass zumindest ein Teil der Hornhaut an dem Funktionselement 12 anliegt. Der Bereich, an dem die Hornhaut 19 anliegt, kann als Applanationslinse bzw. Applanationsplatte 11 ausgebildet sein. Die Vorrichtung umfasst eine Mehrzahl von Unterdruckzuführungen 20, 22, 24 (in Figur 1 ist nur eine erkennbar), die an einer oder je an einer Saugpumpe oder an einer Saugpumpe mit drei separaten Regelungseinrichtungen bzw. drei separaten Regelungsventilen angeschlossen sind. Dadurch kann in dem Saugbereich 4, dem Saugbereich 10 und dem Saugbereich 6 ein unterschiedlicher Unterdruck erzeugt werden. Im Saugbereich 10 kann ein hoher Unterdruck eingestellt werden, damit das Funktionselement 12 fest an dem Saugring 2 angeordnet ist. Im Saugbereich 4 und/oder im Saugbereich 6 kann ein niedrigerer Unterdruck eingestellt werden, damit das Auge 18 nicht verletzt wird. Der Operateur kann ferner vor der Operation lediglich einen Unterdruck im Saugbereich 10 anlegen, um das Funktionselement 12 am Saugring 2 anzuordnen. Beim Positionieren des Saugrings 2 an dem Auge 18 des Patienten kann im Saugbereich 4 ein Unterdruck erzeugt werden, um den Saugring 2 am Auge 18 sicher anzuordnen. Schließlich kann ein Unterdruck im dritten Saugbereich 6 erzeugt werden, damit die Hornhaut 19 des Auges 18 sicher am Funktionselement 12 angeordnet ist. Es ist jedoch auch denkbar, zuerst dem Saugring 2 am Auge 18 anzuordnen und anschließend ein Vakuum im zweiten Saugbereich 10 zu erzeugen, um das Funktionselement 12 anzuordnen.

Die erfindungsgemäße Vorrichtung vermeidet eine Verletzung des Auges, da im Saugbereich 4 ein anderer Unterdruck eingestellt werden kann als im zweiten Saugbereich 10 und/oder im Saugbereich 6. Ferner schafft die vorliegende Erfindung eine Redundanz, da der Saugring 2 sowohl durch den Saugbereich 4 als auch durch den Saugbereich 6 am Auge gehalten wird.

Der Saugbereich 4 ist als umlaufende Nut im Saugring 2 ausgebildet. Es sind jedoch auch andere Ausgestaltungen möglich, beispielsweise eine Mehrzahl von umlaufenden Nuten, eine oder mehrere umlaufende oder nicht umlaufende Einbuchtungen oder eine Mehrzahl von Öffnungen. Der Saugbereich 10 ist durch zwei kreisförmig umlaufende Nuten an der Vorderseite oder Oberseite des Saugring 2 ausgebildet. Auch hier sind die zuvor bezüglich des Saugbereiches 4 beschriebenen Ausgestaltungen möglich. Der Saugbereich 6 wirkt bei der ersten Ausführungsform im Übergangsbereich vom Saugring 2 zum Funktionselement 12. Dadurch kann sichergestellt werden, dass ein Teil der Hornhaut 19 an die Oberfläche des Funktionselementes 12 gesaugt wird.

Das Funktionselement 12 lässt sich einfach wechseln, ohne dass spezielle Werkzeuge und/oder aufwändige Bedienschritte dafür erforderlich sind. Das Funktionselement 12 kann im Einsatz einfach durch ein anderes ausgetauscht werden, das beispielsweise ein anderes optisches Element 11, Applanationselement 11 und/oder einen anderen Anschlussbereich 13 umfasst.

Figur 2a zeigt eine zweite Ausführungsform der Erfindung, wobei gleiche Bezugszeichen wie in Figur 1 gleiche Elemente bezeichnen. Bezüglich der Ausgestaltung der Saugbereiche 4, 6, 10 sowie der Unterdruckzuführungen wird auf die Beschreibung der Ausführungsform gemäß Figur 1 verwiesen.

Im Gegensatz zur Ausführungsform nach Figur 1 wird bei der Ausführungsform nach Figur 2a ein Halterungselement 14 durch einen Unterdruck im Saugbereich 10 am Saugring 2 angeordnet. Das Halterungselement 14 kann ein beliebiges Element sein, an dem weitere Elemente angeordnet oder befestigt werden können. Das Halterungselement 14 umfasst an seinem dem Saugring 2 zugewandten Bereich eine kreisförmig umlaufende Ausnehmung, in der ein optisches Element 16 angeordnet ist. Das optische Element 16 kann als Applanationselement oder als Applanationslinse ausgebildet sein. Das Halterungselement 14 umfasst auch einen (nicht gezeigten) Anschlussbereich, mit dem das Halterungselement mit der Optik eines Lasersystems gekoppelt werden kann.

Durch den Unterdruck im Saugbereich 6 wird die Hornhaut 19 an das am Halterungselement 14 angeordnete optische Element 16 gesaugt.

Figur 2b zeigt eine dritte Ausführungsform der Erfindung, die der zweiten Ausführungsform ähnelt. Im Gegensatz zur zweiten Ausführungsform ist bei der dritten Ausführungsform Saugbereich 10' in dem Funktionselement 14' statt am Saugring 2' angeordnet. Über die Unterdruckzuführung 22' wird ein Unterdruck in dem Saugbereich 10' erzeugt. Die übrigen Ausgestaltungen der Augensaugeinrichtung 2' und des Funktionselementes 14' entsprechen den Ausgestaltungen der Augensaugeinrichtung 2 und des Funktionselementes 14 der zweiten Ausführungsform.

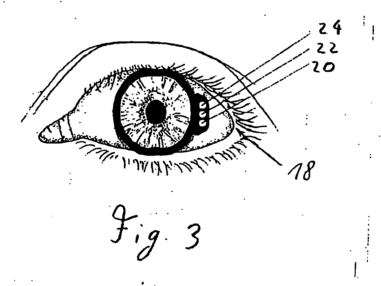

Figur 3 zeigt eine Frontansicht des Saugrings ohne aufgesetztes Funktionselement im Einsatz.

Der Saugring umfasst eine Unterdruckzuführung 20, die mit dem Saugbereich 4 verbunden ist, eine Unterdruckzuführung 22, die mit dem Saugbereich 10 verbunden ist, und eine Unterdruckzuführung 24, die mit dem Saugbereich 6 verbunden ist. Der Saugring muss nicht im Wesentlichen kreisrund sein. Er kann beispielsweise auch als elliptischer Ring oder Polygonring ausgebildet sein.

Figuren 4 und 5 zeigen ein Vergleichsbeispiel mit einem Saugring 116, einem Halterungselement 102, einem Applanationselement 104, einer Zuführeinrichtung 110 mit zumindest einer Unterdruckleitung sowie einer Stromversorgung und einer Signalverbindung für zumindest ein Messmittel. An der Unterseite des Saugrings 116 befindet sich ein erster Saugbereich 128, mit dem der Saugring 116 gegenüber dem Auge 122 fixiert wird. Der Saugring 116 kann über einen zweiten Saugbereich 124, in dem ein Unterdruck herrscht, das Halterungselement 102 fixieren. Das Halterungselement 102 fixiert über einen dritten, optionalen Saugbereich 126 das Applanationselement 104. Durch das Applanationselement 104 wird die Strahlung eines (nicht gezeigten) Lasersystems, beispielsweise eines Femtosekunden-Lasersystems, eingekoppelt. Das Applanationselement kann auch als Linse ausgebildet sein. Das Applanationselement 104 liegt an seinem unteren Ende an der Hornhaut des Auges 122 an, wodurch die Position der Hornhaut festgelegt wird.

An der Oberseite des Halterungselementes 102 ist eine Mehrzahl von mechanischen Führungen 106 zum Ankoppeln des Strahlenganges des Lasers und/oder eines Verschlussmechanismus ausgebildet. Die mechanischen Führungen 106 können einen Kraftsensor (nicht dargestellt) umfassen. Ferner kann der Kraftsensor an den mechanischen Führungen angeordnet werden, wobei die Optik des Lasers in diesem Fall an die Kraftsensoren gekoppelt wird. Der Kraftsensor kann beispielsweise ein piezoelektrischer Sensor sein oder er kann mittels Dehnungsmessstreifen aufgebaut sein.

An dem Applanationselement 104 können Messmittel zum Messen des Augeninnendruckes angeordnet sein. Die Messmittel können durch mikroelektromechanische Systeme (MEM-Systeme) bereitgestellt werden. Am Rand des Applanationselementes 104 befindet sich eine Mehrzahl von Fasersensoren 108 zum Durchführen eines Spektroskopieverfahrens, beispielsweise im nahen Infrarotbereich, und/oder zum Durchführen eines Verfahrens zur Bestimmung der Lichtstreuung. Mit diesen Fasersensoren können Eigenschaften der Hornhaut, beispielsweise deren Wassergehalt, bestimmt werden. Durch die Bestimmung der Lichtstreuung kann auch die Transparenz der Hornhaut bestimmt werden. Hierzu ist ein separater Transparenzsensor 114 vorgesehen, der beispielsweise durch Bestimmung der Lichtstreuung der Hornhaut deren Transparenz ermittelt. Eine mechanische Spektroskopieeinrichtung 112 ermittelt, beispielsweise aufgrund mechanischer Resonanz, die biomechanischen Eigenschaften der Hornhaut. Über die Zuführeinrichtung 110 wird der Vorrichtung Unterdruck zugeführt. Ferner wird mit der Zuführeinrichtung 110 eine Stromversorgung gewährleistet, und die Messsignale der Messmittel werden an eine (nicht gezeigte) Auswerteeinrichtung über ein elektrisches Kabel, eine Glasfaser und/oder Funk übertragen. An dem Applanationselement 104 und dem Halterungselement 102 können Kontaktelemente vorgesehen werden, um elektrische Signale zu übertagen und eine Stromversorgung bereitzustellen.

An dem Halterungselement 102 sind ferner Positionsmessmittel 120 vorgesehen, um die Position des Saugrings 116 bzw. des Halterungselementes 102 im Raum zu bestimmen. Die Positionsmessmittel 120 können beispielsweise akustische oder optische Sensoren sein, die die Position gegenüber einer Referenzgeometrie bestimmen. Ferner kann es sich bei den Positionssensoren 120 um rein passive Sensoren handeln, die einen von einem Referenzort ausgestrahlten Strahl empfangen, wobei auf Basis des empfangenen Signals die Position des Saugrings 116 bzw. des Halterungselementes 102 bestimmt wird. Ebenso können die Positionsmessmittel 120 rein aktive Elemente sein, die einen optischen oder akustischen Strahl aussenden, der von einer entsprechenden Empfangseinrichtung an einem Referenzort empfangen wird, wobei auf Basis des empfangenen Signals die Position der Positionsmessmittel 120 im Raum bestimmt wird. An dem Halterungselement 102 können auch Referenzmarken angeordnet sein, die von einer im Raum angeordneten Kamera erfasst werden. Ebenso können im Inneren der Augensaugeinrichtung optische Sensoren angeordnet sein, die die Position des Auges erfassen. Mit Hilfe der erfassten Position der Augen gegenüber dem Saugring 116 und der über die Positionsmessmittel 120 erfassten Position des Saugringes im Raum kann die Position des Auges 122 im Raum bestimmt werden.

Die Mittel zum Bestimmen der Position des Saugringes im Raum können mit einer (nicht gezeigten) Positionssteuerung einer Behandlungsliege oder einer (nicht gezeigten) Positionssteuerung eines Lasersystems gekoppelt werden.

Der Saugring 116 berührt im Einsatz das Auge. In den Saugring 116 ist ein Kraftsensor 118 integriert, um die auf das Auge wirkende Kraft zu messen, woraus der Augeninnendruck bestimmt werden kann. Im Saugring116 kann eine Mehrzahl von Kraftsensoren 118 angeordnet sein. Dadurch kann ein Kraftprofil entlang dem Umfang des Saugrings 116 erstellt werden, wodurch das Hornhautprofil ermittelt werden kann und demgemäss der Augeninnendruck genauer ermittelt werden kann.

Der Saugring 116 oder das Halterungselement 102 kann ferner einen vierten Saugbereich (nicht gezeigt) umfassen, der den Bereich zwischen dem Applanationselement und der Hornhaut evakuiert, damit die Hornhaut des Auges 122 sicher an dem Applanationselement 104 anliegt.

Der Saugring 116 und das Halterungselement 102 können integral ausgebildet sein oder kraft- oder formschlüssig verbunden sein.

Die vorliegende Erfindung hat den Vorteil, dass sie eine Redundanz schafft, da einerseits der Saugring an das Auge gesaugt wird und andererseits ein Teil der Hornhaut an ein Funktionselement oder ein daran angeordnetes Element gesaugt wird. Ferner hat die Erfindung den Vorteil, dass der Operateur vor, während und/oder nach der Operation ohne Verwendung eines aufwändig zu bedienenden Werkzeuges ein Funktionselement, beispielsweise eine Linse, eine Applanationslinse oder einen Anschlussbereich für die Optik eines Lasersystems, einfach wechseln kann. Die Erfindung hat ferner den Vorteil, dass zum Anordnen des Funktionselementes keine mechanischen Befestigungselemente erforderlich sind, was dazu beiträgt, die Augensaugeinrichtung zu miniaturisieren und deren Gewicht zu reduzieren. Da keine mechanischen Befestigungselemente erforderlich sind, kann die Augensaugeinrichtung aus weniger Bauteilen und einfacher aufgebaut werden, wodurch der Operateur eine bessere Sicht auf das Operationsfeld erhält und das Ausfallsrisiko reduziert wird.

Es versteht sich, dass jeder der Saugbereiche in eine Mehrzahl von autarken Saugbereichen aufgeteilt werden kann, wodurch eine weitere Redundanz mit erhöhter Sicherheit entsteht.

Die vorliegende Erfindung hat den Vorteil, dass sie vor, während und/oder nach einer Operation Messdaten des Auges liefert. Die Erfindung liefert Messdaten bezüglich des Augeninnendrucks, der Position des Saugringes im Raum, der mechanischen Kraft, die auf ein Auge wirkt, Messdaten der Hornhaut, beispielsweise deren Wassergehalt, deren biomechanische Eigenschaften und deren Transparenz. Ferner müssen keine zusätzlichen Messgeräte bereitgestellt werden, um das Auge während einer Operation zu überwachen.

Die Erfindung wurde mittels mehrerer Ausführungsformen beschrieben. Der Fachmann versteht, dass die Merkmale und Merkmalskombinationen der verschiedenen Ausführungsformen kombiniert werden können.

## Patentansprüche

1. Vorrichtung für die Augenchirurgie, umfassend
- einen auf ein zu behandelndes Auge aufsetzbaren Saugring (2) mit einem ersten Saugbereich (4), der dazu ausgebildet ist, durch Unterdruckerzeugung in dem ersten Saugbereich den Saugring an das Auge (18) anzusaugen, und
- ein an den Saugring ankoppelbares Funktionselement (14) mit einem optischen Element (16) zur Anlage der Hornhaut des Auges, wobei der Saugring einen weiteren Saugbereich (6) aufweist, welcher dazu ausgebildet ist, durch Unterdruckerzeugung in dem weiteren Saugbereich das Funktionselement an den Saugring anzusaugen, **dadurch gekennzeichnet, dass** der weitere Saugbereich dazu ausgebildet ist, durch Unterdruckerzeugung in dem weiteren Saugbereich die Hornhaut an das optische Element anzusaugen.

2. Vorrichtung nach Anspruch 1, **dadurch gekennzeichnet, dass** das Funktionselement (14) einen Anschlussbereich zur Ankopplung an ein Lasersystem aufweist.

3. Vorrichtung nach Anspruch 1 oder 2, **dadurch gekennzeichnet, dass** das Funktionselement (14) ein Halterungselement mit einer Ausnehmung ist, in Welcher das optische Element (16) angeordnet ist.

4. Vorrichtung nach einem der Ansprüche 1 bis 3, **dadurch gekennzeichnet, dass** das optische Element eine Linse ist.

5. Vorrichtung nach einem der Ansprüche 1 bis 3, **dadurch gekennzeichnet, dass** das optische Element eine Platte ist.

6. Vorrichtung nach einem der vorhergehenden Ansprüche, **dadurch gekennzeichnet, dass** der Saugring (2) eine mit dem ersten Saugbereich (4) verbundene erste Unterdruckzuführung (20) sowie eine mit dem weiteren Saugbereich (6) verbundene zweite Unterdruekzuführung (24) aufweist.

7. Vorrichtung nach einem der vorhergehenden Ansprüche, **dadurch gekennzeichnet, dass** der Unterdruck in dem weiteren Saugbereich (6) unabhängig von dem Unterdruck in dem ersten Saugbereich (4) einstellbar ist.

8. Vorrichtung nach einem der vorhergehenden Ansprüche, **gekennzeichnet durch** mindestens ein an dem Saugring (116) oder/und an dem Funktionselement (102) angeordnetes Messmittel (120) zur Messung einer Eigenschaft des Auges und/oder des Saugrings bezüglich der Umgebung.

9. Vorrichtung nach Anspruch 8, **dadurch gekennzeichnet, dass** zumindest eines der Messmittel (120) als Positionsmessmittel zur Messung der Position des Saugrings (116) oder/und des Funktionsetements (102) im Raum ausgebildet ist

10. Vorrichtung nach Anspruch 9, **dadurch gekennzeichnet, dass** das Positionsmessmittel (120) dazu ausgelegt ist, mit Positionierungsmitteln für eine Behandlungsliege und/oder Positionierungsmitteln für eine Laserstrahlung zusammenzuwirken.

11. Vorrichtung nach einem der Ansprüche 8 bis 10, **dadurch gekennzeichnet, dass** das Messmittel (120) zum leichteren Desinfizieren und/oder Sterilisieren hermetisch abgeschlossen ist.

12. Vorrichtung nach einem der Ansprüche 8 bis 11, **dadurch gekennzeichnet, dass** das Messmittel (120) dazu ausgelegt ist, ermittelte Messdaten zu übertragen.

13. Vorrichtung nach Anspruch 12, **dadurch gekennzeichnet, dass** das Messmittel (120) ferner eine Kommunikationseinrichtung umfasst.

## Claims

1. Apparatus for eye surgery, comprising:
- a suction ring (2) adapted for being placed onto an eye to be treated and having a first suction portion (4) configured to suck the suction ring to the eye (18) by generating a vacuum in the first suction portion; and
- a function member (14) adapted for coupling to the suction ring and having an optical element for contacting the cornea of the eye, the suction ring comprising a further suction portion (6) configured to suck the function member to the suction ring by generating a vacuum in the further suction portion;
**characterised in that** the further suction portion is configured to suck the cornea to the optical element by generation of a vacuum in the further suction portion.

2. Apparatus of claim 1, **characterised in that** the function member (14) comprises a connection portion for coupling to a laser system.

3. Apparatus of claim 1 or 2, **characterised in that** the function member (14) is a support member having a recess in which the optical element (16) is accommodated.

4. Apparatus of any one of claims 1 to 3, **characterised in that** the optical element is a lens.

5. Apparatus of any one of claims 1 to 3, **characterised in that** the optical element is plate.

6. Apparatus of any one preceding claim, **characterised in that** the suction ring (2) comprises a first vacuum supply (20) connected to the first suction portion (4) and a second vacuum supply (24) connected to the further suction portion (6).

7. Apparatus of any one preceding claim, **characterised in that** the vacuum in the further suction portion (6) is adjustable independent of the vacuum in the first suction portion (4).

8. Apparatus of any one preceding claim, **characterised by** at least one measuring means (120) provided on at least one of the suction ring (116) and the function member (102), for measuring a property of at least one of the eye and the suction ring with respect to the environment.

9. Apparatus of claim 8, **characterised in that** at least one of the measuring means (120) is designed as a position measuring means for measuring the spatial position of at least one of the suction ring (116) and the function member (102).

10. Apparatus of claim 9, **characterised in that** the position measuring means (120) is configured to cooperate with at least one of positioning means for a treatment chair and positioning means for a laser radiation.

11. Apparatus of any one of claims 8 to 10, **characterised in that** the measuring means (120) is hermetically sealed facilitating disinfection and/or sterilization.

12. Apparatus of any one of claims 8 to 11, **characterised in that** the measuring means (120) is configured to transmit obtained measurement data.

13. Apparatus of claim 12, **characterised in that** the measuring means (120) further comprises a communication device.

## Revendications

1. Dispositif pour la chirurgie de l'oeil, comprenant
- un anneau d'aspiration (2) applicable sur un oeil à traiter et comportant une première zone d'aspiration (4) conçue pour faire tenir par aspiration l'anneau d'aspiration sur l'oeil (18) en générant une dépression dans ladite première zone d'aspiration,
- et un élément fonctionnel (14) pouvant être couplé à l'anneau d'aspiration et pourvu d'un élément optique (16) pour l'applanation de la cornée de l'oeil,
l'anneau d'aspiration présentant une zone d'aspiration supplémentaire (6) conçue pour faire tenir par aspiration l'élément fonctionnel (14) sur l'anneau d'aspiration en générant une dépression dans ladite zone d'aspiration supplémentaire, et **caractérisé en ce que** la zone d'aspiration supplémentaire est conçue pour aspirer la cornée contre l'élément optique en générant une dépression dans la zone d'aspiration supplémentaire.

2. Dispositif selon la revendication 1, **caractérisé en ce que** l'élément fonctionnel (14) présente une zone de raccordement pour le couplage à un système laser.

3. Dispositif selon la revendication 1 ou 2, **caractérisé en ce que** l'élément fonctionnel (14) consiste en un élément de maintien pourvu d'une réservation dans laquelle est disposé l'élément optique (16).

4. Dispositif selon l'une des revendications 1 à 3, **caractérisé en ce que** l'élément optique consiste en une lentille.

5. Dispositif selon l'une des revendications 1 à 3, **caractérisé en ce que** l'élément optique consiste en une plaque.

6. Dispositif selon l'une des revendications précédentes, **caractérisé en ce que** l'anneau d'aspiration (2) présente une première conduite à dépression (20) reliée à la première zone d'aspiration (4) ainsi qu'une seconde conduite à dépression (24) reliée à la zone d'aspiration supplémentaire (6).

7. Dispositif selon l'une des revendications précédentes, **caractérisé en ce que** la dépression dans la zone d'aspiration supplémentaire (6) est réglable indépendamment de la dépression dans la première zone d'aspiration (4).

8. Dispositif selon l'une des revendications précédentes, **caractérisé par** au moins un moyen de mesure (120) disposé sur l'anneau d'aspiration (116) ou/et sur l'élément fonctionnel (102) et servant à mesurer une propriété de l'oeit et/ou de l'anneau d'aspiration par rapport à l'environnement.

9. Dispositif selon la revendication 8, **caractérisé en ce qu'**au moins un des moyens de mesure (120) est conçu en tant que moyen de mesure de position pour mesurer la position dans l'espace de l'anneau d'aspiration (116) ou/et de l'élément fonctionnel (102).

10. Dispositif selon la revendication 9, **caractérisé en ce que** le moyen de mesure de position (120) est conçu pour coopérer avec des moyens de positionnement pour une table de soin et/ou des moyens de positionnement pour un rayonnement laser.

11. Dispositif selon l'une des revendications 8 à 10, **caractérisé en ce que** le moyen de mesure (120) est fermé de manière hermétique pour une désinfection et une stérilisation simplifiées.

12. Dispositif selon l'une des revendications 8 à 11, **caractérisé en ce que** le moyen de mesure (120) est conçu pour transmettre des données de mesure saisies.

13. Dispositif selon la revendication 12, **caractérisé en ce que** le moyen de mesure (120) comprend en outre un dispositif de communication.
